(19) Europäisches Patentamt
European Patent Office
Office européen des brevets

(11) **EP 1 972 928 B1**

(12) **EUROPÄISCHE PATENTSCHRIFT**

(45) Veröffentlichungstag und Bekanntmachung des Hinweises auf die Patenterteilung:
**19.09.2012 Patentblatt 2012/38**

(51) Int Cl.:
*G01N 21/64* (2006.01)    *C12Q 1/04* (2006.01)
*G01N 33/38* (2006.01)

(21) Anmeldenummer: **08005275.6**

(22) Anmeldetag: **20.03.2008**

(54) **Vorrichtung und Verfahren zur zerstörungsfreien Prüfung der biostatischen bzw. bioziden Eigenschaften einer photokatalytischen Oberflächenbeschichtung**

Device and method for non-destructive testing of biostatic and/or biocidal properties of a photocatalytic surface coating

Dispositif et procédé de vérification non destructive des caractéristiques biostatiques ou biocides d'un revêtement de surface photocatalytique

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **21.03.2007 DE 102007013471**
**12.07.2007 DE 102007032506**

(43) Veröffentlichungstag der Anmeldung:
**24.09.2008 Patentblatt 2008/39**

(73) Patentinhaber: **ERLUS AKTIENGESELLSCHAFT**
**84088 Neufahrn (DE)**

(72) Erfinder:
• **Bahnemann, Detlef Prof. Dr**
**30826 Garbsen (DE)**

• **Gast, Eduard Dr**
**84559 Kraiburg (DE)**
• **Stoll, Alexander Dr**
**85354 Freising (DE)**
• **Ackerhans, Carsten Dr**
**84061 Ergoldsbach (DE)**
• **Messal, Constanze Dr, rer. nat.**
**18146 Rostock (DE)**

(74) Vertreter: **Louis Pöhlau Lohrentz**
**Patentanwälte**
**Postfach 30 55**
**90014 Nürnberg (DE)**

(56) Entgegenhaltungen:
**WO-A-02/055729    DE-B3-102004 052 764**
**GB-A- 2 386 946    US-A1- 2004 243 318**

## Beschreibung

Die Erfindung betrifft ein Verfahren zur zerstörungsfreien Prüfung einer photokatalytischen Oberflächenbeschichtung durch Messung der Lebensfähigkeit von Mikroorganismen sowie eine Vorrichtung dazu.

Photokatalytische Beschichtungen werden auf keramischen Formkörpern, wie beispielsweise Dachziegeln, eingesetzt, um Verschmutzung und Bewuchs der Dachoberfläche mit Mikroorganismen, wie Algen und Flechten, zu verhindern.

Derartige Beschichtungen sind mit geringer Schichtdicke im μm-Bereich oder nm-Bereich ausgebildet. Sie sind farblos und visuell nicht sichtbar. Zur Messung der Schichtdicke sind zerstörende Verfahren bekannt, wie die Auswertung des mikroskopischen Schliffes einer dem Bauelement entnommenen Probe. Es sind auch zerstörungsfreie chemische Verfahren bekannt, die die Reaktion einer auf die photokatalytische Oberfläche aufgebrachten Testsubstanz bei Bestrahlung mit UV-Licht auswerten.

Die DE 10 2004 052 764 beschreibt ein Verfahren, bei welchem chemische Testsubstanzen auf den Dachziegel aufgebracht und anhand der stofflichen Veränderungen der Testsubstanzen unter UV-Licht-Bestrahlung die photokatalytische Aktivität der Dachziegel bestimmt wird.

WO02/055729A1 offenbart ein Verfahren und eine Vorrichtung zum Nachweis von Mikroorganismen auf Oberflächen wie Mauerwerk. Die zugrundeliegende Aufgabe besteht darin, festzustellen, ob Mikroorganismen auf einer Oberfläche vorhanden sind oder nicht. Bei dem Verfahren wird die Oberfläche sowie ein etwaig vorhandener Mikroorganismus mit einer Lichtquelle bestrahlt, die eine Wellenlänge beinhaltet, die bei Einstrahlung auf etwaig vorhandenem Mikroorganismus, eine Photodetektion des Mikroorganismus zulässt und/oder der Mikroorganismus durch eine Farb- oder Lichtreaktion nachgewiesen wird.

Ferner beschreibt die DE 10 2004 037 555 A1 ein Verfahren, bei welchem die von der photokatalytischen Oberfläche des Bauelements reflektierte Strahlung zur Bestimmung der photokatalytischen Aktivität des Dachziegels verwendet wird.

Aufgabe der vorliegenden Erfindung ist es, ein Verfahren und eine Vorrichtung zur Prüfung von Bauelementen mit einer photokatalytischen Oberflächenbeschichtung bereitzustellen, welche eine schnelle, zerstörungsfreie Prüfung der Oberflächenbeschichtung erlauben, die auch in den Herstellungsprozess integriert werden können.

Erfindungsgemäß wird diese Aufgabe durch ein Verfahren zur zerstörungsfreien Prüfung einer photokatalytischen Oberflächenbeschichtung von keramischen Bauelementen, beispielsweise Dachziegeln, gelöst, wobei eines oder mehrere Bauelemente auf einer Transportvorrichtung folgende Verfahrensschritte durchlaufen:

a) Aufbringen von lebenden Mikroorganismen auf einen Prüfbereich der photokatalytischen Oberflächenbeschichtung eines keramischen Bauelements,

b) Aufbringen mindestens eines Farbstoffes auf den Prüfbereich, welcher Farbstoff so ausgebildet ist, dass die Lebensfähigkeit der Mikroorganismen an der Farbe und/oder Lichtabsorption und/oder Fluoreszenz des Farbstoffs ablesbar ist,

c) Bestrahlen des Prüfbereiches mit UV-Licht, und

d) Detektieren der Farbe und/oder Lichtabsorption und/oder Fluoreszenz des Prüfbereiches mit einer Detektionsvorrichtung,

wobei die Reihenfolge der Schritte vorzugsweise wie folgt ist: a),b),c),d) oder b),a),c),d) oder a),c),b),d) und wobei der Schritt a) und der Schritt b) und/oder der Schritt c) und der Schritt d) jeweils in der angegebenen Reihenfolge oder gleichzeitig ausgeführt werden können.

Die einzelnen Verfahrensschritte können in unterschiedlicher Reihenfolge durchgeführt werden. Die Mikroorganismen und der Farbstoff können zusammen oder getrennt aufgebracht werden. Das Bestrahlen mit UV-Licht und das Detektieren können gleichzeitig oder nacheinander durchgeführt werden.

Zwischen dem Schritt c) und d) sollten weniger als 30 bis 90 sec liegen. Vorzugsweise wird der Prüfbereich solange mit UV-Licht bestahlt bis die Detektion durchgeführt wird.

Bevorzugte Weiterbildungen des erfindungsgemäßen Verfahrens sind in den Unteransprüchen 2 bis 16 angegeben.

Bei einer Ausführungsform ist vorgesehen, dass der Farbstoff an die Mikroorganismen gekoppelt ist und/oder der Farbstoff in den Mikroorganismen enthalten ist und/oder der Farbstoff und die Mikroorganismen als Gemisch vorliegen. Bei einer Ausführungsform koppelt der Farbstoff an die Zellmembran der Mikroorganismen und/oder an die DNA der Mikroorganismen und/oder an Kohlenhydrate und/oder Proteine der Mikroorganismen.

Bei einer Ausführungsform enthalten die Mikroorganismen bereits einen natürlichen Farbstoff, beispielsweise Chlorophyll.

Bei einer Ausführungsform des erfindungsgemäßen Verfahrens handelt es sich um ein kontinuierliches Verfahren.

Unter "kontinuierlichem Verfahren" im Sinne der Erfindung ist vorzugsweise ein Verfahren zu verstehen, bei dem mindestens ein Bauelement auf einer Transportvorrichtung die oben beschriebenen Verfahrensschritte durchläuft, wobei die Transportvorrichtung zwischen den einzelnen Verfahrensschritten angehalten werden kann und/oder die Bauelemente in Lagern zwischen den einzelnen Verfahrensschritten zwischengelagert werden können. Vorzugsweise durchläuft in dem kontinuierlichen Verfahren das mindestens eine Bauelement die Verfahrensschritte ohne die Transportvorrichtung anzuhalten.

Bei einer Ausführungsform werden die Verfahrensschritte a) bis d) in einem kontiniuierlichen Inline-Verfahren durchgeführt. Unter "kontinuierlichem Inline-Verfahren" im Sinne der Erfindung ist vorzugsweise ein Verfahren zu verstehen, bei dem eine Vielzahl von Bauelementen auf einer Transportvorrichtung die oben beschriebenen Verfahrensschritte durchläuft, wobei die Transportvorrichtung zwischen den einzelnen Verfahrensschritten angehalten werden kann und/oder die Bauelemente in Lagern zwischen den einzelnen Verfahrensschritten zwischengelagert werden können. Vorzugsweise durchlaufen die Bauelemente bei dem kontinuierlichen Inline-Verfahren die Verfahrensschritte ohne die Transportvorrichtung anzuhalten.

Vorzugsweise werden die Bauelemente unmittelbar nach der Herstellung der photokatalytischen Oberflächenbeschichtung der Prüfung unterzogen, wobei vorzugsweise die Herstellung der photokatalytischen Beschichtung und die Prüfung der photokatalytischen Oberflächenbeschichtung in einem kontinuierlichen oder kontinuierlichen Inline-Verfahren erfolgen.

"Lebensfähigkeit" und/oder "lebend" im Sinne der Erfindung bedeutet vorzugsweise, dass die Mikroorganismen einen funktionierenden Stoffwechsel und/oder eine intakte Zellmembran aufweisen.

Bei einer Ausführungsform wird bei der Bestimmung der Lebensfähigkeit der Mikroorganismen die Zahl der toten und/oder der lebenden Mikroorganismen, bezogen auf eine Positivkontrolle und/oder Negativkontrolle, durch Messung des Absorptionsspektrum bestimmt. Vorzugsweise erfolgt diese Messung nicht für einzelne Zellen sondern über einen bestimmten Flächenbereich, d.h. integral.

Bei einer Ausführungsform wird bei der Bestimmung der Lebensfähigkeit der Mikroorganismen das Verhältnis von lebenden zu toten Mikroorganismen bestimmt. Vorzugsweise beträgt das Verhältnis von lebenden zu toten Mikroorganismen 0,25 oder weniger. Weiter bevorzugt beträgt das Verhältnis von lebenden zu toten Mikroorganismen 0,1 oder weniger.

Unter toten Mikroorganismen sind vorzugsweise Mikroorganismen zu verstehen, die keine intakte Zellmembran aufweisen. Bei einer Ausführungsform wird bei der Prüfung des funktionierenden Stoffwechsels beispielsweise die Photosyntheseaktivität und/oder enzymatische Aktivität und/oder mikrobielle Aktivität überprüft.

Unter mikrobieller Aktivität im Sinne der Erfindung ist die Stoffwechselgeschwindigkeit der Mikroorganismen zu verstehen.

Unter inaktiven Mikroorganismen sind vorzugsweise Mikroorganismen zu verstehen, die eine Photosyntheseaktivität und/oder enzymatische Aktivität und/oder mikrobielle Aktivität von weniger als 20 %, vorzugsweise weniger als 5%, bezogen auf Mikroorganismen aufweisen, die keiner photokatalytisch aktiven Oberflächenbeschichtung ausgesetzt wurden. Die verbleibenden Mikroorganismen können als aktive Mikroorganismen bezeichnet werden.

Vorzugsweise werden Mikroorganismen bei dem erfindungsgemäßen Verfahren in Schritt a) eingesetzt, die sich in der exponentiellen Wachstumsphase befinden. Die exponentielle Wachstumsphase kann auch als log-Phase bezeichnet werden. Vorzugsweise wird der auf den Schritt a) folgende Schritt erst 4 bis 12 h, vorzugsweise 6 bis 8 h, nach dem Aufbringen der Mikroorganismen in Schritt a) durchgeführt, damit die Mikroorganismen anwachsen können.

Bei einer Ausführungsform werden die Bauelemente auf der Transportvorrichtung stichprobenartig überprüft. Stichprobenartig bedeutet, dass nur ein Teil der Bauelemente, beispielsweise jedes 100ste Bauelement, überprüft wird.

Der Prüfbereich kann die gesamte Oberfläche oder Teile der Oberfläche der Bauelemente umfassen. Sofern der Prüfbereich des Bauelements nach dessen Einbau noch sichtbar ist, d.h. sich z.B. farblich und/oder strukturell von seiner Umgebung abhebt, ist vorzugsweise vorgesehen, den Prüfbereich in den nach dem Einbau nicht sichtbaren Bereich des Bauelements zu legen. Beispielsweise ist vorgesehen, den Prüfbereich bei Dachziegeln in einem Bereich anzuordnen, der in der Verlegung des Dachziegels in einer Dacheindeckung nicht sichtbar ist. Ein solcher Bereich ist beispielsweise der Kopffalzbereich, der durch den Fußfalzbereich des firstseitig benachbarten Dachziegels überdeckt ist.

Bei einer Ausführungsform ist die Transportvorrichtung ein Transportband oder eine Vorrichtung, die sich aus mehreren Transportbändern zusammensetzt, wobei vorzugsweise die Bauelemente direkt von einem Transportband auf das nächste befördert werden.

Überraschenderweise ermöglicht das erfindungsgemäße Verfahren die zerstörungsfreie Prüfung einer photokatalytischen Oberflächenbeschichtung vorzugsweise in einem Zeitraum von 15s bis 120s, weiter bevorzugt in einem Zeitraum von 15s bis 60s, am meisten bevorzugt in einem Zeitraum von 15s bis 30s.

Bei einer Ausführungsform des erfindungsgemäßen Verfahrens erfolgt die Detektion in Schritt d) in kürzester Zeit von vorzugsweise in 1s bis 45s, am meisten bevorzugt in 1s bis 10s, wobei die Schritte a), b) und c) vorzugsweise innerhalb von 14s bis 119s, vorzugsweise in 14 bis 29 s, durchgeführt werden.

Die kurze Reaktionszeit erlaubt es, die Methode in einen kontinuierlichen Produktionsprozess, vorzugsweise kontinuierlichen Inline-Produktionsprozess einzubinden. Das erfindungsgemäße Verfahren kann daher als Qualitätsprüfung der photokatalytischen Oberflächenbeschichtung im Herstellungsprozess der Bauelemente eingesetzt werden, ohne dass der Herstellungsprozess für die Prüfung unterbrochen werden muss.

Unter zerstörungsfreier Prüfung im Sinne der Erfindung ist zu verstehen, dass bei der Prüfung keine Veränderung der Bauelemente eintritt, die die Bauelemente als Produkt unbrauchbar macht. Unter zerstörungsfreiem Prüfverfahren wird insbesondere verstanden, das das Bauelement nicht zerstört wird und/oder eine Probe entnommen wird. Vorzugsweise wird das Bauelement durch das Prüfverfahren nicht sichtbar beeinträchtigt und/oder die im Prüfverfahren verwendeten Farbstoffe werden, vorzugsweise durch Wasser, nach Schritt d) abgewaschen.

Bei einer Ausführungsform handelt es sich bei den in Schritt a) lebenden Mikroorganismen um Bakterien und/ oder Protozoen und/oder Pilze und/oder Algen. Vorzugsweise handelt es sich bei den Mikroorganismen um UVresistente Mikroorganismen. UV-resistent bedeutet in diesem Zusammenhang, dass bei Bestrahlung mit UV-Licht, insbesondere UV-A-Licht, nach 1 min mindestens 95 %, vorzugsweise 99% der Mikroorganismen, die vor der Bestrahlung lebten, noch am Leben sind. Das bei der Bestrahlung verwendete UV-Licht, insbesondere das UV-A-Licht, weist vorzugsweise eine Strahlungsleistung von weniger als 40 W/m$^2$, weiter bevorzugt eine Strahlungsleistung aus einem Bereich von 5 bis 40 W/m$^2$, besonders bevorzugt aus einem Bereich von 8 bis 15 W/m$^2$, auf. Das UV-A-Licht weist beispielsweise eine Wellenlänge aus einem Bereich von 320 bis 400 nm auf.

Vorzugsweise werden Algen, insbesondere Grün- und/oder Blaualgen verwendet. Bei den Blaualgen handelt es sich um sogenannte Cyano-Bakterien. Als Grünlage kann beispielsweise "Stichcoccus bacillaris" und/oder "Prasiola crispa", "Coeno Cystes" und/oder "Chlorella spec." verwendet werden.

Bei einer Ausführungsform des erfindungsgemäßen Verfahrens werden Schritt a) und b) gleichzeitig ausgeführt, d.h. die Mikroorganismen und der mindestens eine Farbstoff werden gleichzeitig aufgetragen. Die Mikroorganismen und der Farbstoff können als Gemisch aufgetragen werden und/oder der Farbstoff ist im Mikroorganismus enthalten und/oder der Farbstoff liegt an die Mikroorganismen gekoppelt vor. Die Kopplung des Farbstoffs kann beispielsweise durch Kopplung von farbmarkierten Gensonden durchgeführt werden. Falls der Farbstoff in dem Mikroorganismus enthalten ist, kann es sich um einen Farbstoff handeln, der bereits natürlich im Mikroorganismus vorkommt, wie beispielsweise Chlorophyll.

Vorzugsweise werden die Mikroorganismen und/oder der Farbstoff in Form von Lösungen und/oder Suspensionen aufgebracht.

Die Lebensfähigkeit der Mikroorganismen kann zum einen dadurch bestimmt werden, dass zu einem bestimmten Zeitpunkt des Prüfverfahrens das Verhältnis der lebenden Mikroorganismen zu den toten Mikroorganismen bestimmt wird. Hierzu wird zu einem bestimmten Zeitpunkt, nachdem die Mikroorganismen- und Farblösung auf das Bauelement aufgebracht wurde und das Bauelement mit UV-Licht bestrahlt wurde, das Verhältnis der lebenden zu den toten Zellen bestimmt. Vorzugsweise beträgt das Verhältnis von lebenden zu toten Zellen 0,25 oder weniger.

Bei einer anderen Ausführungsform kann die Lebensfähigkeit der Mikroorganismen dadurch bestimmt werden, dass zu einem bestimmten Zeitpunkt des Prüfverfahrens das Verhältnis der Mikroorganismen mit einem funktionierenden Stoffwechsel, d.h. aktiven Mikroorganismen zu den inaktiven Mikroorganismen bestimmt wird. Vorzugsweise beträgt das Verhältnis von aktiven zu inaktiven Mikroorganismen 0,25 oder weniger.

Bei einer anderen Ausführungsform kann die Lebensfähigkeit der Zellen dadurch bestimmt werden, dass die Zahl der lebenden Zellen und/oder der aktiven Zellen zu zwei unterschiedlichen Zeitpunkten bestimmt wird. Um die Lebensfähigkeit der Zellen zu zwei unterschiedlichen Zeitpunkten bestimmen zu können, wird in Schritt c) der Prüfbereich von einem Zeitpunkt $t_0$ bis zu einem Zeitpunkt $t_2$ mit UV-Licht bestrahlt. Das Detektieren der Farbe und/oder der Lichtabsorption und/oder der Fluoreszenz des Prüfbereichs mit einer Detektionsvorrichtung in Schritt d) wird zum Zeitpunkt $t_1$ und zum Zeitpunkt $t_2$ durchgeführt, wobei der Zeitpunkt $t_1$ zwischen dem Zeitpunkt $t_0$ und $t_2$ liegt. Bei einer Ausführungs-form beträgt der Zeitraum von $t_0$ bis $t_1$ 2 bis 20 s. Bei einer Ausführungsform beträgt der Zeitraum von $t_1$ bis $t_2$ 5 bis 60 s.

Die Zahl der in diesem Zeitraum zwischen den zwei Zeitpunkten $t_1$ und $t_2$ getöteten und/oder inaktivierten Zellen der Mikroorganismen bezogen auf die Gesamtzahl der Zellen, welche auch als Sterbegeschwindigkeit bezeichnet werden kann, kann als Nachweis für die Qualität der photokatalytischen Beschichtung verwendet werden. Bei dieser Ausführungsform des erfindungsgemäßen Verfahrens erfolgt das Detektieren in Schritt d) zu zwei verschiedenen Zeitpunkten nach dem Durchführen der Schritte a), b) und c). Vorzugsweise wird nachdem der letzte der Schritte a), b) und c) durchgeführt wurde eine erste Messung zum Zeitpunkt $t_1$ innerhalb von 0,5 bis 2 s durchgeführt und die zweite Messung zum Zeitpunkt $t_1$ wird 1 bis 5 s später durchgeführt.

Als Negativkontrolle 1 für das erfindungsgemäße Prüfverfahren kann ein Bauelement ohne photokatalytische Beschichtung verwendet werden. Als Negativkontrolle 2 für das erfindungsgemäße Prüfverfahren kann ein Bauelement verwendet werden, dessen photokatalytisch aktive Oberflächenbeschichtung in dem Prüfverfahren nicht mit UV-Licht aktiviert wird. Als Negativkontrolle 3 für das erfindungsgemäße Prüfverfahren kann ein Bauelement verwendet werden, auf welches keine Mikroorganismen aufgetragen werden.

Als Positivkontrolle für das erfindungemäße Prüfverfahren kann ein Bauelement eingesetzt werden, bei dem die Qualität und Vollständigkeit der photokatalytischen Oberflächenbeschichtung bereits durch ein anderes Prüfverfahren überprüft und als ausreichend befunden wurde.

Bei dem erfindungsgemäßen Verfahren kann die Lebensfähigkeit der Mikroorganismen mit der Qualität der photokatalytischen Oberflächenbeschichtung korreliert werden. Ferner kann mit dem erfindungsgemäßen Verfahren überprüft werden, ob mindestens 90 %, vorzugsweise 100% der Oberfläche des Bauelements mit der photokatalytischen Oberflächenbeschichtung beschichtet wurde. Mit dem erfindungsgemäßen Verfahren kann insbesondere überprüft werden, ob die Oberfläche des photokatalytisch beschichteten Bauelements superhydrophil und vorzugsweise der Kontaktwinkel der photokatalytisch beschichteten Dachziegel unter 5 ° liegt.

Falls mehr als 40 %, vorzugsweise mehr als 70 %, besonders bevorzugt mehr als 90 %, der Zellmembranen bei der Prüfung mit dem erfindungsgemäßen Verfahrens, bezogen auf die Gesamtzahl der Zellen, zerstört werden bzw. zerstört sind, liegt vorzugsweise ein Bauelement mit einer ausreichenden Oberflächenbeschichtung vor.

Bei einer Ausführungsform des erfindungsgemäßen Verfahrens werden die Farbe und/oder Lichtabsorption und/oder Fluoreszenz des Farbstoffs in Form von Prüfwerten einem Computer zur Auswertung und/oder Prozesssteuerung des Herstellungs- und/oder Prüfungsverfahrens zugeführt.

Bei einer Ausführungsform des erfindungsgemäßen Verfahrens liegt der Wellenlängenbereich des UV-Lichtes in Schritt c) in einem Bereich von 280 nm bis 390 nm, vorzugsweise von 315 nm bis 380 nm. Bei einer Ausführungsform wird eine schmalbandige UV-Lichtquelle als UV-Bestrahlungsquelle verwendet, vorzugsweise mit einer Wellenlänge in einem Wellenlängenmaximumbereich von 350 bis 370 nm. Die Bestrahlungsintensität liegt bei einer Ausführungsform in einem Bereich von 5 mW/cm$^2$ bis 15 mW/cm$^2$. Vorzugsweise wird der Prüfbereich 5s bis 45s, vorzugsweise 15 s bis 30 s, mit UV-Licht bestrahlt.

Vorzugsweise erfolgt die Detektion mit einer Detektionsvorrichtung, die zum Nachweis von Licht, insbesondere Licht eines speziellen Spektralbereiches, geeignet ist. Hierzu wird vorzugsweise ein Filter, vorzugsweise ein sogenannter Kantenfilter, verwendet, der nur Licht einer bestimmten Wellenlänge durchläßt.

Beispielsweise wird selektiv Licht aus dem gelb-, rot- und/oder grün-Bereich detektiert. Insbesondere wird Licht aus einem Wellenlängenbereich von 575 - 585 nm (gelb), 650 - 750 nm (rot) und/oder 490 - 575 nm (grün) detektiert.

Bei einer Ausführungsform des erfindungsgemäßen Verfahrens wird als Detektionsvorrichtung eine optische Sensorvorrichtung, vorzugsweise eine Bilderfassungsvorrichtung, verwendet. Beispielsweise kann als Bilderfassungsvorrichtung der Detektionsvorrichtung eine elektronische Kamera verwendet werden. Bei einer Ausführungsform umfasst die Detektionsvorrichtung 2 Sensoren zum Nachweisen von 2 verschiedenen Spektralbereichen.

Bei einer bevorzugten Ausführungsform wird die Detektion durch Nachweis von Fluoreszenzstrahlung durchgeführt. Bei dieser Ausführungsform wird als Detektionsvorrichtung vorzugsweise ein Fluorometer verwendet. Vorzugsweise umfasst das Fluorometer eine Lichtquelle im Blaubereich zur Anregung der Fluoreszenz und einen Messsensor mit einem Wellenlängenbereich von grünem und rotem Licht. Der Farbstoff wird vorzugsweise mit Licht, ausgewählt aus einem Wellenlängenbereich von 400 bis 600, vorzugsweise 400 bis 550 nm, angeregt. Die Detektion erfolgt vorzugsweise mit Licht ausgewählt aus einem Wellenlängenbereich von 450 bis 700 nm, weiter bevorzugt mit Licht ausgewählt aus einem Wellenlängenbereich von 450 bis 650 nm. Die Fluoreszenz kann neben herkömmlichen Detektionsmethoden auch durch Epifluoreszenz, Laser-induzierter Fluoreszenz (LIF) oder durch Puls-Amplituden-modulierte Fluoreszenz (PAM) bestimmt werden. Bei den herkömmlichen Detektionsmethoden wird die Eigenfluoreszenz bestimmt. Bei den Verfahren Epifluoreszenz, Laser-induzierter Fluoreszenz (LIF) oder durch Puls-Amplituden-modulierte Fluoreszenz (PAM) wird durch Bestrahlung die Fluoreszenz in den Zellen angeregt. Bei der Epifluoreszenzmikroskopie wird die Probe vorzugsweise von oben bestrahlt.

Die PAM-Diagnostik macht sich vorzugsweise zu nutze, dass eine klare quantitative Beziehung zwischen der Fluoreszenzquantenausbeute und der photosynthetischen Energieumwandlung existiert. Vorzugsweise werden Mikroorganismen, insbesondere Bakterienzellen oder Algen, eingesetzt, die sich in der exponentiellen Wachstumsphase befinden. Vorzugsweise beträgt die optische Dichte der verwendeten Mikroorganismensuspension, insbesondere Bakteriensuspension oder Algensuspension, bei 550 nm $OD_{550}$ = 0,5 bis 0,6. Diese Messung ist besonders für Messungen über einen längeren Zeitraum von mindestens 7 Tagen, vorzugsweise 30 bis 50 Tagen, geeignet. Bei einer Ausführungsform des Puls-Amplituden-Modulierten-Fluorometers werden gepulstes Anregungslicht (Messlicht), kontinuierliches, Photosynthese treibendes Licht (aktinisches Licht) und synchronisierte Sättigungsblitze verwendet. Eine bevorzugte Ausführungsform für die PAM-Diagnostik ist nachfolgend unter Punkt a) bis n) erläutert

a) Die Messung der relativen Fluoreszenzquantenausbeute wird hierzu im dunkel-adaptierten Zustand oder im hell-adaptierten Zustand der Zellen durchgeführt.

b) Für die Messung im dunkel-adaptierten Zustand muss die Probe beispielsweise mindestens 10 min, vorzugsweise 10 bis 15 min, bei Dunkelheit verbleiben. Dies kann beispielsweise dadurch geschehen, dass die Probe durch einen abgedunkelten Transporttunnel transportiert wird.

c) Für die Messung im helladaptierten Zustand muss die Probe vorher beispielsweise mindestens 10 min, vorzugsweise 10 bis 15 min, bei Helligkeit vorzugsweise von 100 bis 1000 Lux, weiter bevorzugt von 700 bis 800 Lux, verbleiben, wobei vorzugsweise die Wellenlänge des eingestrahlten Lichtes in einem Bereich von 360 bis 390 nm liegt.

d) Vorzugsweise wird die Photosynthese nach dem Erreichen des dunkel- oder helladaptierten Zustandes mit kontinuierlichem Licht (aktinisches Licht), ausgewählt aus einem Wellenlängenbereich von 460 bis 480 nm mit einer Intensität von 1100 bis 1300 $\mu$mol quanta/m$^2$s PAR angetrieben.

e) Der Sättigungsblitz weist vorzugsweise einen Wellenlängenbereich, ausgewählt aus dem Bereich von 460 bis 480 nm, mit einer Intensität von 2200 bis 2400 $\mu$mol quanta/m$^2$s PAR auf. Vorzugsweise dauert der Sättigungsblitz 10 $\mu$s bis 100 ms, weiter bevorzugt 1 bis 10 ms.

f) Vorzugsweise wird die Fluoreszenz mit gepulstem Licht (Messlicht), ausgewählt aus einem Wellenlängenbereich von 460 bis 480 nm, mit einer Intensität von 0,4 bis 0,6 $\mu$mol quanta/m$^2$s PAR angeregt.

g) In dem dunkel-adaptierten Zustand wird beispielsweise zunächst die sogenannte minimale Fluoreszenzquantenausbeute $F_o$ gemessen. Anschließend wird die dunkeladaptierte Probe zusätzlich mit einem Sättigungsblitz beleuchtet, der vorzugsweise sämtliche Photosynthese II Reaktionszentren in den Zellen blockiert. In diesem angeregten Zustand wird die maximale Fluoreszenzquantenausbeute $F_m$ mit Messlicht gemessen.

h) Aus $F_0$ und $F_m$ lässt sich die relative Fluoreszenzquantenausbeute $\Phi_F$ im dunkeladaptierten Zustand wie folgt berechnen:

$$\Phi_F = (F_m - F_0)/F_m$$

i) Da die Berechnung der relativen Fluoreszenzquantenausbeute aus dem Verhältnis von 2 Fluoreszenzgrößen gebildet wird, wirken sich die Variabilitätsfaktoren wie Messlichtintensität, Probengröße und Abstand zwischen Probe und Detektor nicht auf das Messergebnis aus, solange die Parameter zwischen der $F_0$- und $F_m$-Messung nicht geändert werden.

j) Die relative Fluoreszenzquantenausbeute im dunkeladaptierten Zustand $\Phi_F$ ist bei gestressten oder toten Pflanzen kleiner als bei ungestressten oder lebenden Pflanzen. Die relative Fluoreszenzquantenausbeute im dunkeladaptierten Zustand $\Phi_F$ liegt bei ungestressten und/oder lebenden Pflanzen in einem Bereich vom 0,7 bis 0,8. Die relative Fluoreszenzquantenausbeute im dunkeladaptierten Zustand $\Phi_F$ liegt bei ungestressten und/oder lebenden Pflanzen, die auf Baustoffen wachsen, in einem Bereich vom 0,6 bis 0,45. Bei photokatalytisch beschichteten Dachziegeln liegt die relative Fluoreszenzquantenausbeute im dunkeladaptierten Zustand $\Phi_F$ in einem Bereich von 0,1 bis 0,55.

k) Alternativ wird im hell-adaptierten Zustand beispielsweise zunächst die minimale Fluoreszenzquantenausbeute $F_o'$ im helladaptierten Zustand im natürlichen Licht mit Messlicht gemessen. Anschließend wird die helladaptierte Probe zusätzlich mit einem Sättigungsblitz beleuchtet, der vorzugsweise sämtliche Photosynthese II Reaktionszentren in den Zellen blockiert. In diesem angeregten Zustand wird die maximale Fluoreszenzquantenausbeute $F_m'$ im natürlichen Licht mit Messlicht gemessen.

l) Aus $F_o'$ und $F_m'$ lässt sich die relative Fluoreszenzquantenausbeute im helladaptierten Zustand $\Phi_F'$ wie folgt berechnen:

$$\Phi_F' = (F_m' - F_o')/F_m'$$

m) Da die Berechnung der relativen Fluoreszenzquantenausbeute im helladaptierten Zustand $\Phi_F'$ aus dem Verhältnis von 2 Fluoreszenzgrößen gebildet wird, wirken sich die Variabilitätsfaktoren wie Messlichtintensität, Probengröße und Abstand zwischen Probe und Detektor auch nicht auf das Messergebnis im helladaptierten Zustand aus, solange die Parameter zwischen der $F_0$'- und $F_m$'-Messung nicht geändert werden.

n) Die relative Fluoreszenzquantenausbeute ist daher auch im helladaptieren Zustand bei gestressten oder toten Pflanzen kleiner als bei ungestressten oder lebenden Pflanzen. Die relative Fluoreszenzquantenausbeute im helladaptieren Zustand $\Phi_F$' liegt bei ungestressten und/oder lebenden Pflanzen in einem Bereich vom 0,7 bis 0,8. Die relative Fluoreszenzquantenausbeute im helladaptieren Zustand $\Phi_F$' liegt bei ungestressten und/oder lebenden Pflanzen, die auf Baustoffen wachsen, in einem Bereich vom 0,6 bis 0,45. Bei photokatalytisch beschichteten Dachziegeln liegt die relative Fluoreszenzquantenausbeute im helladaptieren Zustand $\Phi_F$' in einem Bereich von 0,1 bis 0,55.

Es sind Ausführungen vorgesehen, die alle Optionen a) bis n) enthalten oder auch nur eine Auswahl dieser Optionen.

Zur Berechnung der Strahlungsleistungsdichte P kann folgende Berechnung herangezogen werden:

Die Strahlungsleistungsdichte P ist von der Wellenlänge $\lambda$ abhängig.

Es gilt:

$$E = h\nu = hc/\lambda$$

$\nu$ = Frequenz
E = Energie
c = Lichtgeschwindigkeit
N = Avogadrozahl = $6,022*10^{23}$/mol

Mit h = $6,626*10^{-34}$ Js (Planck), c = $2,99*10^8$ m/s, N = $6,022*10^{23}$/mol und einem Lichtquant = hv folgt für die Intensität I :

$$I\ [\mu mol\ Lichtquanten\ /\ m^2\ s] = I\ [\mu mol\ h\nu\ /\ m^2\ s] = I\ [\mu mol\ hc/\lambda\ m^2\ s] = I/\lambda\ [10^{-6}$$
$$mol * 6,626*10^{-34}\ Js * 2,99*10^8\ m/s * 1/m^2\ s] = I/\lambda * 19,81174*10^{-32}\ [mol\ J\ /\ m\ s].$$

Für die Strahlungsleistungsdichte P [W/m$^2$] gilt dann:

$$P = I * N = I/\lambda * 19,81174*10^{-32}\ [mol\ J\ /\ m\ s] * 6,022*10^{23}\ /mol = I/\lambda *$$
$$119,306298*10^{-9}\ [J\ /\ m\ s]\ , also$$

$$P = 1,19306298*10^{-7} * I/\lambda\ \ [W\ /\ m]$$

bei Licht von 470 nm = $4,7*10^{-7}$ m ergibt sich also eine intensitätsabhängige Strahlungsleistungsdichte von

$$P(470) = 0,254\ * I\ [W\ /\ m^2],$$

oder bei 350nm von

$$P(350) = 0,341\ * I\ [W\ /\ m^2].$$

Anstelle des Begriffs "Lichtquant" kann auch der Begriff "quanta" verwendet werden.

Bei einer Ausführungsform enthalten die Mikroorganismen bereits einen Farbstoff dessen Farbe und/oder Lichtabsorption und/oder Fluoreszenz sich in Abhängigkeit von der Lebensfähigkeit der Mikroorganismen ändert.

Beispielsweise können Grünalgen durch Fluoreszenzlicht bei einer Wellenlänge von 685 nm nachgewiesen werden. Die Anregungswellenlänge beträgt vorzugsweise ungefähr 550 nm, falls ein Detektionsverfahren eingesetzt wird, das auf der Anregung von Fluoreszenz beruht

Bei einer Ausführungsform des erfindungsgemäßen Verfahrens werden in Schritt b) mehrere Farbstoffe verwendet.

Bei einer Ausführungsform des erfindungsgemäßen Verfahrens wird ein Farbstoff verwendet, der in Bereichen des Bauelements mit lebenden Mikroorganismen eine andere Farbe und/oder ein anderes Fluoreszenz- und/oder ein anderes Absorptionsspektrum aufweist als mit toten und/oder inaktiven Mikroorganismen. Vorzugsweise handelt es sich um organische Farbstoffe, welche vorzugsweise aromatische, polycyclische Verbindungen sind, die konjugierte Doppelbindungen aufweisen.

Beispielsweise verändert der Farbstoff sein Absorptions- und/oder Fluoreszenzspektrum dadurch, dass durch die zerstörte Zellmembran Farbstoff in die toten Zellen eindringt und dort vorzugsweise an die DNA der Mikroorganismen bindet.

Bei einer Ausführungsform wird als Farbstoff ein Fluoreszenzfarbstoff verwendet.

Bei einer Ausführungsform werden die Farbstoffe aus der Gruppe, bestehend aus Triphenylfarbstoffen, Antracenfarbstoffen, Azofarbstoffen, Cyaninfluoreszenzfarbstoffen, Acridinfarbstoffen, Carbonylfarbstoffen und Mischungen davon, ausgewählt.

Vorzugsweise werden als Farbstoff

Chlorophyll und/oder
2-{2-[(3-Dimethylamino-propyl)-propylamino]-1-phenyl-1*H*-chinolin-4-ylidenmethyl}-3-methyl-benzothiazol-3-ium-Kation (SybrGreenII® der Firma
Invitrogen) und/oder
Acridinorange (3,6-Bis-(dimethylamino)-acridin-hydrochlorid Zinkchlorid-Doppelsalz) und/oder
DAPI (4',6-Diamidino-2-phenylindol) und/oder
CalcofluorWhite® und/oder
Sytox® (Invitrogen) und/oder
Bromthymolblau (3',3"-Dibromthymolsulfonphthalein) und/oder
Baclight® (Invitrogen) und/oder
Methylenblau (3,7-Bis(dimethylamino)-phenothiaziniumchlorid (Methylthioniniumchlorid))
und/oder
Propidiumiodid
und/oder
LIVE/DEAD® BacLight(™) Bacterial Viability Kit (catalog number L-7012,
Molecular Probes, Inc., Eugene, OR.EUB338)
und/oder
EUB338
und/oder
CTC (Cyanoditolyltetrazolium chloride)
und/oder
Cell Tracker
und/oder
Green™
und/oder
CMAC
und/oder
L-Leu (7-Amino-4-chloromethylcoumarinleucinamid)
und/oder
Indigocarmin (4-(Dimethylamino)-azobenzol-4'-sulfonsäure Natriumsalz (Methylorange) und/oder 3,3'-Dioxo-2,2'-biindolinyliden-5,5'-disulfonsäure
Dinatriumsalz)

verwendet.

Vorzugsweise wird als Farbstoff Bromthymolblau, SYBR Green I®, Sytox®, Acrdinorange und/oder DAPI eingesetzt.

Bei einer Ausführungsform werden die Bauelemente in einer gegen Fremdlicht abgeschirmten Bestrahlungsstation mit UV-Licht bestrahlt. Bei einer Ausführungsform des erfindungsgemäßen Verfahrens befindet sich die gesamte Transportvorrichtung in einem gegen Fremdlicht abgeschirmten Prüftunnel.

Bei einer Ausführungsform des erfindungsgemäßen Verfahrens gibt die Detektionsvorrichtung ein Echtfarbenbild oder ein Falschfarbenbild aus.

Bei einer Ausführungsform des erfindungsgemäßen Verfahrens wird das Prüfverfahren mittels einer Transportvorrichtung durchgeführt, welche sich mit einer Geschwindigkeit von 5 m/s bis 18 m/s vorwärts bewegt.

Bei einer Ausführungsform des erfindungsgemäßen Verfahrens wird die relative Lage des Bauelementes auf der Transportvorrichtung mit Lagesensoren bestimmt.

Bei einer Ausführungsform des erfindungsgemäßen Verfahrens gibt der Computer, dem die Prüfwerte zugeführt werden, Steuerbefehle für eine Sortiervorrichtung für die Bauelemente aus. Vorzugsweise werden die Steuerbefehle aus dem Soll-Ist-Vergleich der Prüfwerte eines oder mehrerer Kontrollbauelemente(s) und des zu prüfenden Bauelementes abgeleitet. Als Kontrollbauelemente können die oben beschriebenen Positiv- und Negativkontrollen verwendet werden.

Bei einer Ausführungsform des erfindungsgemäßen Verfahrens werden die Steuerbefehle aus der Zeitdifferenz zwischen dem Beginn der UV-Bestrahlung des Bauelements und dem Eintreten des Farbumschlags des Prüfbereichs abgeleitet.

Bei einer Ausführungsform des erfindungsgemäßen Verfahrens werden die Mikroorganismen und/oder die/der Farbstoff(e) von der Oberfläche des Dachziegels, vorzugsweise mit Wasser, nach Schritt d) abgewaschen.

Die Aufgabe wird ferner durch eine Vorrichtung (10) zur zerstörungsfreien Prüfung einer photokatalytischen Oberflächenbeschichtung eines Bauelements (20) gelöst, umfassend

eine Mikroorganismenauftragsstation (30) mit einem Vorratsgefäß, in welchem Mikroorganismen aufgenommen sind (30v), welches vorzugsweise temperbar ist, und eine Vorrichtung zum Aufbringen der Mikroorganismen (30a) auf einen Prüfbereich (90) eines Bauelements ,
eine Färbestation (40) mit einem Vorratsgefäß (40v), in welchem ein oder mehrere Farbstoffe aufgenommen sind und eine Vorrichtung zum Aufbringen der Farbstoffe (40a) auf den Prüfbereich (90), und
eine Bestrahlungsstation (50) mit einer UV-Bestrahlungsquelle (50u), die über dem Prüfbereich (90) angeordnet ist,
eine Detektionsstation (60) mit einer über dem Prüfbereich (90) angeordneten Detektionsvorrichtung, wobei
die Vorrichtung eine Transportvorrichtung (80) umfasst, auf der ein oder mehrere Bauelemente (20) durch die Verfahrensstationen transportiert werden.

Bevorzugte Weiterbildungen der erfindungsgemäßen Vorrichtung sind in den Unteransprüchen 18 bis 27 angegeben.

Bei einer Ausführungsform sind die Mikroorganismenauftragsstation und/oder die Färbestation und/oder die Bestrahlungsstation und/oder die Detektionsstation in einer gemeinsamen Station zusammengefasst.

Bei einer Ausführungsform ist die Detektionsstation zum qualitativen und/oder quantitativen Nachweis der Farbe und/oder Absorptionsspektrum und/oder Fluoresenzspektrum und/oder deren Änderungen ausgebildet.

Die Vorratsgefäße zum Aufbringen der Mikroorganismen bzw. des Farbstoffs sind durch Leitungen mit den Vorrichtungen zum Aufbringen der Mikroorganismen bzw. des Farbstoffs verbunden.

Bei dem Bauelement kann es sich um Dachziegel und/oder Kaminbestandteile mit photokatalytischer Beschichtung handeln.

Das erfindungsgemäße Verfahren kann auch als Prüfverfahren bezeichnet werden.

Die erfindungsgemäße Vorrichtung ist vorzugsweise derart ausgestaltet, dass die oben beschriebenen Verfahrensvarianten ausgeführt werden können.

Bei einer Ausführungsform ist das Vorratsgefäß der Färbestation und/oder die Vorrichtung zum Auftragen der Farbstofflösung und/oder die Färbestation lichtundurchlässig ausgebildet.

Bei einer Ausführungsform ist die Detektionsvorrichtung mit einem Computer (70) mit Auswertungssoftware verbunden.

Bei einer Ausführungsform sind die Bauelemente auf einer Transportvorrichtung abgelegt, die integraler Bestandteil der Herstellungsvorrichtung ist. Wenn zur Durchführung des Prüfverfahrens ein schrittweiser Transport der Bauelemente vorgesehen ist, kann die Transportvorrichtung mit mindestens einem Zwischenlager ausgebildet sein.

Als Mikroorganismen können Bakterien und/oder Algen und/oder Protozoen und/oder Pilze, wie vorstehend angegeben, verwendet werden. Als Farbstoffe können Farbstoffe verwendet werden, die geeignet sind, um die Lebensfähigkeit der Mikroorganismen nachzuweisen.

Bei dem Photokatalysator handelt es sich um einen Katalysator, der durch UV-Licht aktiviert wird und in diesem aktivierten Zustand die lebenden Mikroorganismen abtötet und/oder deren Wachstum hemmt. Das verringerte Wachstum lässt sich beispielsweise durch die verringerte Stoffwechselaktivität nachweisen. Vorzugsweise handelt es sich bei dem Photokatalysator um Titandioxid und/oder Zinkoxid. Vorzugsweise wird der Titandioxidkatalysator mit UV-Strahlung voraktiviert. Zinkoxid kann auch ohne Bestrahlung eingesetzt werden.

Die Mikroorganismenlösung und die Farbstofflösung können Hilfsstoffe enthalten, die nicht photokatalytisch umgesetzt werden.

Bei einer Ausführungsform ist die Detektionsvorrichtung zum Nachweis einer veränderten Farbe und/oder Fluoreszenz und/oder Absorption ausgebildet. Bei einer Ausführungsform ist die Detektionsvorrichtung als Kamera ausgebildet. Vorzugsweise ist die Detektionsvorrichtung als Fluorometer ausgebildet.

Bei einer Ausführungsform weist die Mikroorganismenauftragsstation und/oder die Färbestation stromabwärts zu der Auftragsstation eine Trockenvorrichtung auf.

Die UV-Bestrahlungsquelle kann als breitbandige UV-Lichtquelle ausgebildet sein, die UV-Licht mit einer Wellenlänge in einem Bereich von 280 nm bis 390 nm abstrahlt, vorzugsweise mit einer Wellenlänge von 315 nm bis 380 nm. Auf diese Weise wird das UV-Strahlungsspektrum der Sonne nachgebildet, insbesondere der für die photokatalytische Wirkung besonders wirksame UV-A-Bereich des Sonnenlichtes. Alternativ kann vorgesehen sein, die UV-Lichtquelle als schmalbandige UV-Lichtquelle auszubilden.

Bei einer Ausführungsform umfasst die Mikroorganismenauftragsstation ein Vorratsgefäß, welches temperierbar ist. Vorzugsweise wird die Temperatur auf einen Bereich eingestellt, in dem sich die Mikroorganismen nicht vermehren, ihre Lebensfähigkeit aber aufrechterhalten wird. Das Vorratsgefäß kann gekühlt und/oder erwärmt werden. Vorzugsweise kann die Temperatur auf 4 bis 8 °C oder 10 bis 15°C eingestellt werden. Vorzugsweise werden Mikroorganismen verwendet, die sich in der exponentiellen Wachstumsphase befinden. Bei einer Ausführungsform wird das Vorratsgefäß in einem Zeitraum von 1 h bis 24 h, vorzugsweise in einem Zeitraum von 2 h bis 6h, mit einer neuen Mikroorganismenlösung befüllt, wobei sich die Mikroorganismen in der exponentiellen Wachstumsphase befinden. Während dieses Zeitraumes kann kontinulierlich oder diskontinuierlich gemessen werden.

Die Vorrichtung zum Aufbringen der Mikroorganismen und/oder Farbstoffe kann als eine automatische Pipette ausgebildet sein. Sie kann aber auch in Art eines Tintenstrahl-Druckkopfes ausgebildet sein, wodurch vorteilhafterweise die Dosierung der Farbstofflösung besonders gut einstellbar ist. Die Vorrichtung zum Aufbringen der Mikroorganismen und/oder Farbstoffe kann auch als Sprühvorrichtung ausgebildet sein.

Weiter kann vorgesehen sein, dass der Computer mit einer Vorrichtung verbunden ist zur Aussonderung fehlerhafter Bauelemente und/oder zur Gruppierung der Bauelemente nach Qualitätsklassen oder/und zur akustischen und/oder optischen Signalisierung und/oder zur Markierung der Bauelemente.

In einer besonders vorteilhaften Ausgestaltung der Erfindung ist vorgesehen, dass die vorstehend genannten Komponenten in eine Prüfkammer (100) integriert sind, so dass die Prüfkammer eine kompakte und abgeschlossene Einheit bildet, die vorteilhafterweise das Gestell für weitere Baugruppen ist.

Bei einer weiteren vorteilhaften Ausgestaltung der Erfindung kann vorgesehen sein, dass die Prüfkammer als kompakter Prüfkopf ausgebildet ist, der alle notwendigen Verfahrensstationen in kompakter und miniaturisierter Ausbildung einschließt. So kann vorgesehen sein, die UV-Bestrahlungsquelle und die Lichtquelle zum Anregen der Fluoreszenz als Leuchtdioden auszubilden und den optischen Sensor als Photodiode bzw. als Photodiodenzeile oder -matrix.

Bei einer Ausführungsform ist die Detektionsstation so ausgestaltet, dass zusätzlich zu dem Messlicht auch noch Sättigungslichtimpulse abgestrahlt werden können.

Bei einer Ausführungsform ist nach der Detektionsstation eine Reinigungsstation zum Reinigen des Bauelements von den Mikroorganismen und/oder dem Farbstoff vorgesehen.

Die Erfindung wird nun anhand der Zeichnung näher erläutert.

Fig. 1    zeigt den schematischen Aufbau eines Ausführungsbeispiels der Vorrichtung zur zerstörungsfreien kontinuierlichen Inline-Prüfung einer photokatalytischen Oberfläche eines keramischen Bauelements;

Fig. 2    zeigt Epifluoreszenzmikroskopie-Aufnahmen einer ohne (Fig 2a) und mit $TiO_2$ beschichteten (Figur 2b) Dachziegelprobe, welche gemäß einem Ausführungsbeispiel des erfindungsgemäßen Verfahrens aufgenommen wurden.

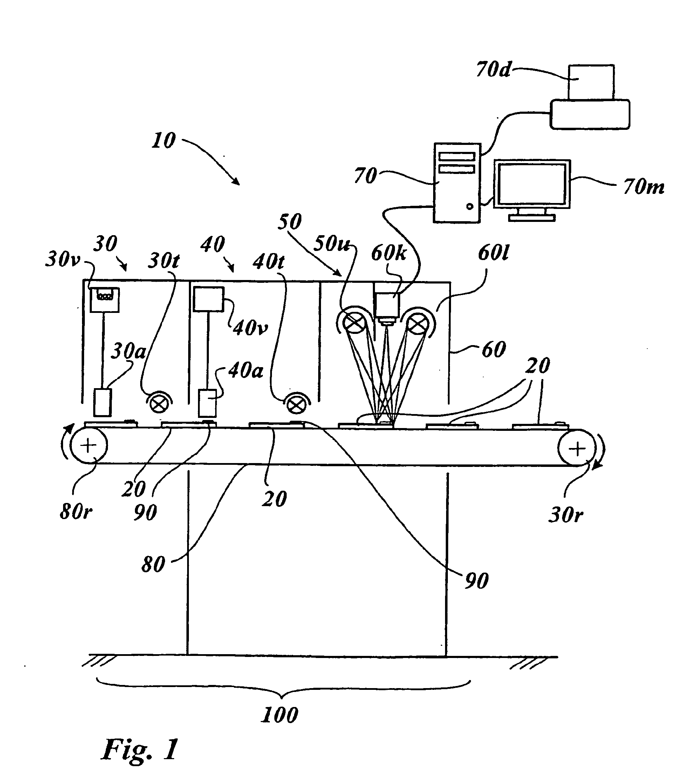

Fig. 1 zeigt eine Prüfvorrichtung (10) zur zerstörungsfreien biologischen kontinuierlichen Inline-Prüfung der photokatalytischen Aktivität einer photokatalytischen Oberfläche eines Bauelements (20). In dem dargestellten Ausführungsbeispiel handelt es sich um die Inline-Prüfung von keramischen Dachziegeln (20).

Die Dachziegel (20) sind auf einem umlaufenden Förderband (80) abgelegt, das von Rollen (80r) angetrieben wird. Das Förderband ist in Fig. 1 von links nach rechts verlaufend dargestellt.

Die Prüfungsvorrichtung umfasst in dem dargestellten Ausführungsbeispiel die folgenden apparativen Einrichtungen: Mikroorganismenauftragsstation (30), Färbestation (40), Bestrahlungsstation (50) und eine Detektionsstation (60). Die einzelnen Stationen sind in dem dargestellten Ausführungsbeispiel in einer Prüfkammer (100) zusammengefasst.

Die Dachziegel (20) durchlaufen zuerst die Mikroorganismenauftragsstation (30). Diese umfasst ein temperierbares Vorratsgefäß (30v), welches die Mikroorganismen enthält, eine Auftragsvorrichtung zum Auftragen der Mikroorganismen (30a), und eine Trockenvorrichtung (30t). Das Vorratsgefäß (30v) ist durch eine Leitung mit der Auftragsvorrichtung zum Auftragen der Mikroorganismen (30a) verbunden.

Anschließend durchlaufen die Dachziegel die Färbestation (40). Diese umfasst ein Vorratsgefäß, welches einen Fluoreszenzfarbstoff enthält (40v), eine Auftragsvorrichtung (40a) und eine Trockenvorrichtung (40t). Das Vorratsgefäß (40v) ist durch eine Leitung mit der Auftragsvorrichtung zum Auftragen des Farbstoffs (40a) verbunden.

Nachfolgend durchlaufen die Dachziegel die Bestrahlungsstation (50). Diese umfasst eine breitbandige UV-Lampe (50u).

Unmittelbar danach durchlaufen die Dachziegel die Detektionsstation (60). Diese umfasst eine Lampe (60l) zum Anregen von Fluoreszenzstrahlung und eine Detektionsvorrichtung, welche als Fluorometer (60k) ausgebildet ist.

Das Fluorometer (60k) umfasst in dem in Fig. 1 dargestellten Ausführungsbeispiel alle Komponenten, um ein digitales Bild, vorzugsweise ein digitales Farbbild, des Dachziegels (20) bereitzustellen und einem Computer (70) zwecks digitaler Bildanalyse zur Verfügung zu stellen.

Der Computer (70) ist in dem dargestellten Ausführungsbeispiel mit einem Monitor (70m) und einem Drucker (70d) verbunden. Auf diese Weise sind die Messwerte online auf dem Schirm des Monitors (70m) sichtbar, mittels des Druckers (70d) protokollierbar und für die Steuerung des Prüfprozesses verwendbar. Bei einer Ausführungsform der erfindungsgemäßen Vorrichtung umfasst diese eine Netzwerkverbindung in eine Betriebsdatenbank für Qualitätsmanagement.

Die Dachziegel (20) werden also an der linken Seite des Förderbandes (80) aufgelegt, durchlaufen nacheinander die Mikroorganismenauftragsstation (30), die Färbestation (40), die Bestrahlungsstation (50) und die Detektionsstation (60) und werden auf der rechten Seite des Förderbandes entnommen.

Beim Durchlauf eines Dachziegels (20) durch die Mikroorganismenauftragsstation (30) wird auf diesen unter der Auftragsvorrichtung (30a) im Prüfbereich (90) eine Mikroorganismenlösung aufgetragen, die in dem dargestellten Beispiel unter der stromabwärts angeordneten Trockenvorrichtung (30t) getrocknet wird.

Beim anschließenden Durchlauf des Dachziegels (20) durch die Färbestation (40) wird dieser unter der Auftragsvorrichtung (40a) im Prüfbereich (90) mit einer Fluoreszenzfarblösung versehen, die in dem dargestellten Beispiel unter der stromabwärts angeordneten Trockenvorrichtung (40t) getrocknet wird.

In der Bestrahlungsstation (50) wird nun dieser mit dem Prüfbereich (90) versehene Dachziegel (20) durch eine UV-Lampe (50u) mit UV-Licht bestrahlt, welche über dem Prüfbereich (90) des Dachziegels angeordnet ist. Dadurch wird auf Grund der photokatalytischen Oberflächenbeschichtung des Dachziegels (20) im Prüfbereich (90) eine photokatalytische Reaktion ausgelöst, wodurch im Prüfbereich (90) die Mikroorganismen sterben und/oder inaktiviert werden. Dies bewirkt eine Änderung des Fluoreszenzspektrums des Fluoreszenzfarbstoffs.

In der Detektionsstation (60) wird mit einer Lampe (60l), welche über dem Prüfbereich des Dachziegels angeordnet ist, die Fluoreszenzstrahlung angeregt. Ein Fluorometer (60k), welches über dem Prüfbereich des Dachziegels angeordnet ist, erzeugt von dem Prüfbereich des Dachziegels (20) ein digitales Bild, aus dem der Computer (70) die Information über die Veränderung des Farbstoffs des Prüfbereichs (90) gewinnt. Das Eintreten des Farbumschlags ist ein Maß für die Beurteilung der Qualität der photokatalytischen Beschichtung des Dachziegels (20).

Es kann vorgesehen sein, dass der Computer (70) mit einer nicht dargestellten Steuerung verbunden ist, welche die Sortierung der Dachziegel (20) in Qualitätsklassen nach dem Verlassen der Detektionsstation (60) steuert, wofür weitere nicht dargestellte Vorrichtungen vorgesehen sein können.

Durch das nachfolgende Beispiel soll die vorliegende Erfindung näher erläutert, aber in keiner Weise beschränkt werden.

**Beispiel**

Eine ohne $TiO_2$ und eine mit $TiO_2$ beschichtete Dachziegelprobe wurde mit der in Figur 1 dargestellten Vorrichtung ohne Verwendung der Trockenvorrichtungen überprüft.

Im ersten Verfahrensschritt a) wurden 1 ml oder 10 ml "Stichococcus bacillaris" als wässerige Suspension (mineralisches Nährstoffmedium) auf einen 4 cm x 4 cm großen Prüfbereich eines Dachziegels aufgetragen und getrocknet. Beispielsweise wurden die Dachziegel mit einem Kaltluftfön bei 30°C getrocknet. Anschließend wurde der Dachziegel 12 h gelagert, damit die Zellen anwachsen. Im zweiten Verfahrensschritt b) wurde eine Farbstofflösung Sytox von der Firma Invitrogen aufgetragen. Im dritten und vierten Verfahrensschritt c) und d) wurden die Dachziegel mit UV-Licht mit einer Wellenlänge von 360 bis 390 nm mit 11 mW/cm$^2$ bestrahlt, unmittelbar danach erfolgte eine Blauanregung mit Licht im Wellenlängenbereich von 400 bis 430 nm.

Bereits nach 30s Bestrahlung war deutlich das veränderte Fluoreszenzspektrum bei der $TiO_2$-beschichteten Dachziegelprobe festzustellen. Bei der beschichteten Dachziegelprobe veränderte sich die Fluoreszenzstrahlung von rot nach grün, wodurch deutlich wurde, dass der "Stichococcus bacillaris" abgetötet wurden. Die Dachziegelprobe des unbeschichteten Dachziegels wies jedoch keine veränderte Fluoreszenzstrahlung auf.

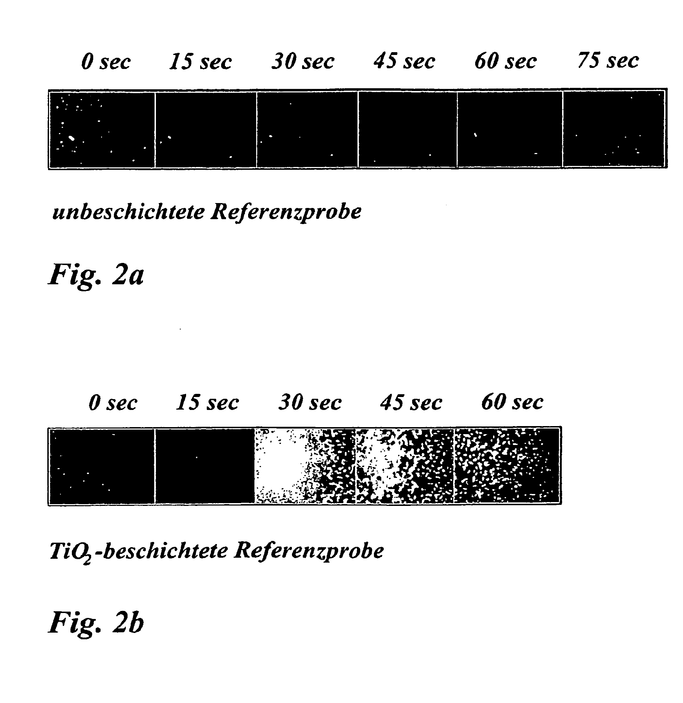

Figur 2 zeigt die Messergebnisse mittels Epifluoreszenzmikroskopie 0s, 15s, 30s, 45s, 60s und 75s nach Beginn der Messung. Die rot-fluoreszierenden lebenden "Stichococcus bacillaris" sind als dunkelgraue Punkte sichtbar, die grün-fluoreszierenden toten"Stichococcus bacillaris" sind als hellgraue Punkte sichtbar. Die Dachziegelprobe ohne photokatalytische Beschichtung weist ein unverändertes Fluoreszenzspektrum über die gesamte Messdauer auf (Fig. 2a). Die Dachziegelprobe mit photokatalytischer Beschichtung weist nach 30 s ein verändertes Fluoreszenzspektrum auf, welches deutlich macht, dass die Algen abgetötet wurden (Fig. 2b).

Die gesamte Messdauer für eine Probe betrug 2 min. Das Experiment zeigt deutlich, dass innerhalb von wenigen Minuten die photokatalytische Beschichtung eines Dachziegels überprüft werden kann. Da bereits nach 30 Sekunden die meisten Bakterien abgestorben sind und die Messung nach 30 Sekunden ein eindeutiges Messergebnis ergibt, zeigt das Beispiel, dass das erfindungsgemäße Messverfahren in weniger als 1 min durchgeführt werden kann.

Bezugszeichenliste

| 10 | Prüfvorrichtung |
| 20 | Bauelement |
| 30 | Mikroorganismenauftragsstation |
| 30a | Vorrichtung zum Aufbringen der Mikroorganismen |
| 30v | temperiertes Vorratsgefäß für Mikroorganismen |
| 30t | Trockenvorrichtung für Mikroorganismen |
| 40 | Färbestation zum Aufbringen des Farbstoffs |
| 40a | Vorrichtung zum Aufbringen des Farbstoffs |
| 40v | Vorratsgefäß für Farbstoff |
| 40t | Trockenvorrichtung für Farbstoff |
| 50 | UV-Bestrahlungseinrichtung mit |
| 50u | UV-Lampe |
| 60 | Detektionsstation |
| 60k | elektronische Kamera |
| 60l | Lampe zum Anregen der Fluoreszenz |
| 70 | Computer |
| 70d | Drucker |
| 70m | Monitor |
| 80 | Förderband |
| 80r | Rolle |
| 90 | Prüfbereich |
| 100 | Prüfkammer |

**Patentansprüche**

1.  Verfahren zur zerstörungsfreien Prüfung einer photokatalytischen Oberflächenbeschichtung von keramischen Bauelementen, beispielsweise Dachziegeln, wobei eines oder mehrere Bauelemente auf einer Transportvorrichtung folgende Verfahrensschritte durchlaufen:

    a) Aufbringen von lebenden Mikroorganismen auf einen Prüfbereich der photokatalytischen Oberflächenbeschichtung eines keramischen Bauelements,
    b) Aufbringen mindestens eines Farbstoffes auf den Prüfbereich, wobei der Farbstoff so ausgebildet ist, dass die Lebensfähigkeit der Mikroorganismen an der Farbe und/oder Lichtabsorption und/oder Fluoreszenz des Farbstoffs ablesbar ist,
    c) Bestrahlen des Prüfbereiches mit UV-Licht, und

d) Detektieren der Farbe und/oder Lichtabsorption und/oder Fluoreszenz des Prüfbereiches mit einer Detektionsvorrichtung,

wobei die Reihenfolge der Schritte vorzugsweise wie folgt ist: a),b),c),d) oder b),a),c),d) oder a),c),b),d) und wobei der Schritt a) und der Schritt b) und/oder der Schritt c) und der Schritt d) jeweils in der angegebenen Reihenfolge oder gleichzeitig ausgeführt werden können.

2. Verfahren nach Anspruch 1, wobei
vorgesehen ist, dass der Farbstoff an die Mikroorganismen gekoppelt ist und/oder
der Farbstoff in den Mikroorganismen enthalten ist
und/oder
der Farbstoff und die Mikroorganismen als Gemisch vorliegen.

3. Verfahren nach Anspruch 1 oder 2, wobei es sich um ein kontinuierliches Verfahren handelt.

4. Verfahren nach einem der vorhergehenden Ansprüche, wobei dem Prüfverfahren gemäß Anspruch 1 die Herstellung der Bauelemente vorausgeht, vorzugsweise unmittelbar vorausgeht, und die Herstellung der Bauelemente und das Prüfverfahren gemäß Anspruch 1 in einem kontinuierlichen Verfahren durchgeführt werden.

5. Verfahren nach einem der Ansprüche 1 bis 4, wobei es sich um ein Inline-Verfahren handelt.

6. Verfahren nach einem der vorhergehenden Ansprüche, wobei die Bauelemente auf der Transportvorrichtung stichprobenartig geprüft werden.

7. Verfahren nach einem der vorhergehenden Ansprüche, wobei es sich bei den lebenden Mikroorganismen um Bakterien und/oder Pilze und/oder Protozoen und/oder Algen handelt.

8. Verfahren nach Anspruch 7, wobei es sich bei den lebenden Mikroorganismen um Grünalgen und/oder Blaualgen handelt.

9. Verfahren nach einem der vorhergehenden Ansprüche, wobei das Verfahren in einem Zeitraum von 15 s bis 120 s, vorzugsweise 15 s bis 60 s, weiter bevorzugt 15 s bis 45 s, durchgeführt wird.

10. Verfahren nach einem der vorhergehenden Ansprüche, wobei das Detektieren zweimal, d.h. zu zwei verschiedenen Zeitpunkten nach der Durchführung der Schritte a), b) und c) erfolgt.

11. Verfahren nach einem der vorhergehenden Ansprüche,
wobei in Schritt d) die Farbe und/oder Lichtabsorption und/oder die Fluoreszenz des Prüfbereichs in Form von Prüfwerten einem Computer zur Auswertung und/oder Prozesssteuerung des Herstellungs- und/oder Prüfverfahrens zugeführt werden.

12. Verfahren nach einem der vorhergehenden Ansprüche,
wobei der Wellenlängenbereich des UV-Lichtes in Schritt c) aus dem Bereich von 280 nm bis 390 nm, vorzugsweise von 315 nm bis 380 nm, ausgewählt ist.

13. Verfahren nach einem der vorhergehenden Ansprüche,
wobei in Schritt b) Farbstoffe verwendet werden, die vorzugsweise aus der
Gruppe, bestehend aus Chlorophyll, Acridinorange, DAPI, CalcoflourWhite, Sytox, Bromthymolblau, Baclight, Methylenblau, Methylorange, Propidiumiodid
EUB338, Cyanoditolyltetrazolium chloride, Cell Tracker, Green, CMAC, 7-Amino-4-chloromethylcoumarinleucinamid
und/oder Indigocarmin und Mischungen davon, ausgewählt sind.

14. Verfahren nach einem der vorhergehenden Ansprüche,
wobei als Negativkontrolle 1 ein Bauelement ohne photokatalytische Beschichtung verwendet wird und/oder
wobei als Negativkontrolle 2 ein Bauelement verwendet wird, dessen photokatalytische aktive Oberflächenbeschichtung nicht in Schritt c) mit UV-Licht aktiviert wird und/oder
wobei als Negativkontrolle 3 ein Bauelement verwendet wird, auf welches in Schritt a) keine Mikroorganismen aufgetragen werden und/oder wobei als Positivkontrolle ein Bauelement verwendet wird, bei dem die Qualität und Vollständigkeit der photokatalytisch aktiven Oberflächenbeschichtung bereits durch ein anderes Prüfverfahren überprüft und als ausreichend befunden wurde.

15. Verfahren nach einem der vorhergehenden Ansprüche 11 bis 14, wobei der Computer, dem die Prüfwerte zugeführt werden, Steuerbefehle für eine Sortiervorrichtung für die Bauelemente ausgibt.

16. Verfahren nach einem der vorhergehenden Ansprüche, wobei die Mikroorganismen und/oder der Farbstoff nach Schritt d) von der Oberfläche der Bauelemente abgewaschen werden.

17. Vorrichtung (10) zur zerstörungsfreien Prüfung einer photokatalytischen Oberflächenbeschichtung eines keramischen Bauelements (20), umfassend
eine Mikroorganismenauftragsstation (30) mit einem Vorratsgefäß, in welchem Mikroorganismen aufgenommen sind (30v), und eine Vorrichtung zum Aufbringen der Mikroorganismen (30a) auf einen Prüfbereich (90) eines Bauelements,
eine Färbestation (40) mit einem Vorratsgefäß (40v), in welchem ein oder mehrere Farbstoffe aufgenommen sind, und eine Vorrichtung zum Aufbringen der Farbstoffe (40a) auf den Prüfbereich (90), und
eine Bestrahlungsstation (50) mit einer UV-Bestrahlungsquelle (50u), die über dem Prüfbereich (90) angeordnet ist,
eine Detektionsstation (60) mit einer über dem Prüfbereich (90) angeordneten Detektionsvorrichtung, wobei
die Vorrichtung eine Transportvorrichtung (80) umfasst, auf der ein oder mehrere Bauelemente (20) durch die Verfahrensstationen transportiert werden.

18. Vorrichtung nach Anspruch 17, wobei
die Mikroorganismenauftragsstation und/oder die Färbestation und/oder die Bestrahlungsstation und/oder die Detektionsstation in einer gemeinsamen Station zusammengefasst sind.

19. Vorrichtung nach Anspruch 17 oder 18, wobei das Vorratsgefäß, in welchem die Mikroorganismen aufgenommen sind, temperierbar ist.

20. Vorrichtung nach einem der Ansprüche 17 bis 19, wobei die Vorrichtung als Vorrichtung zum Durchführen eines kontinuierlichen und/oder kontinuierlichen Inline-Verfahrens ausgestaltet ist.

21. Vorrichtung nach einem der Ansprüche 17 bis 20, welche zur Durchführung des Verfahrens nach einem der Ansprüche 1 bis 16 ausgestaltet ist.

22. Vorrichtung nach einem der Ansprüche 17 bis 21, wobei in der Färbestation das Vorratsgefäß, die Vorrichtung zum Auftragen der Farbstofflösung und/oder die Färbestation lichtundurchlässig ausgebildet sind.

23. Vorrichtung nach einem der Ansprüche 17 bis 22, wobei die Detektionsvorrichtung mit einem Computer (70) mit Auswertungssoftware verbunden ist.

24. Vorrichtung nach einem der Ansprüche 16 bis 23, wobei die Detektionsvorrichtung als Fluorometer ausgebildet ist.

25. Vorrichtung nach einem der Ansprüche 16 bis 24,
wobei die Mikroorganismenauftragsstation und/oder Färbestation stromabwärts zusätzlich eine Trockenvorrichtung (30t/40t) aufweisen.

26. Vorrichtung nach einem der Ansprüche 16 bis 25,
wobei der Computer mit einer Vorrichtung verbunden ist zur Aussonderung fehlerhafter Bauelemente und/oder zur Gruppierung der Bauelemente nach Qualitätsklassen und/oder zur akustischen und/oder optischen Signalisierung und/oder Markierung der Bauelemente.

27. Vorrichtung nach einem der Ansprüche 16 bis 26,
wobei nach der Detektionsstation eine Reinigungsstation zum Reinigen des Bauelements von den Mikroorganismen und/oder dem Farbstoff vorgesehen ist.

**Claims**

1. Method for non-destructive testing of a photocatalytic surface coating of ceramic components, for example roof tiles, wherein one or more components on a transport device are subjected to the following method steps:

   a) application of living microorganisms to a test area of the photocatalytic surface coating of a ceramic component,
   b) application of at least one dye to the test area, the dye being formed in such a way that the viability of the microorganisms can be read from the dye and/or light absorption and/or fluorescence of the dye,
   c) irradiation of the test area with UV light, and
   d) detection of the dye and/or light absorption and/or fluorescence of the test area with a detection device,

   wherein the sequence of the steps is preferably as follows:

   a), b), c), d), or b), a), c), d), or a), c), b), d), and wherein step a) and step b), and/or step c) and step d) can each be carried out in the given sequence or simultaneously.

2. Method according to claim 1, wherein the dye is coupled to the microorganisms and/or the dye is contained in the microorganisms and/or the dye and the microorganisms are provided as a mixture.

3. Method according to claim 1 or 2, wherein the method is a continuous method.

4. Method according to one of the preceding claims, wherein the production of the components precedes the test method according to claim 1, preferably immediately, and the production of the components and the test method according to claim 1 are carried out in a continuous method.

5. Method according to one of claims 1 to 4, wherein the method is an inline method.

6. Method according to one of the preceding claims, wherein the components on the transport device are tested randomly.

7. Method according to one of the preceding claims, wherein the living microorganisms are bacteria and/or fungi and/or protozoa and/or algae.

8. Method according to claim 7, wherein the living microorganisms are green algae and/or blue algae.

9. Method according to one of the preceding claims, wherein the method is carried out in a period of 15 s to 120 s, preferably 15 s to 60 s, more preferably 15 s to 45 s.

10. Method according to one of the preceding claims, wherein detection takes place twice, that is to say at two different moments in time once steps a), b) and c) have been carried out.

11. Method according to one of the preceding claims, wherein in step d) the dye and/or light absorption and/or the fluorescence of the test area are fed to a computer in the form of test values for evaluation and/or process control of the production and/or test method.

12. Method according to one of the preceding claims, wherein the wavelength range of the UV light in step c) is selected from the range of 280 nm to 390 nm, preferably from 315 nm to 380 nm.

13. Method according to one of the preceding claims, wherein in step b) dyes are used which are preferably selected from the group consisting of chlorophyll, acridine orange, DAPI, Calcoflour White, Sytoc, bromothymol blue, BacLight, methylene blue, methyl orange, propidium iodide EUB338, cyanoditolyltetrazolium chloride, cell tracker green, CMAC, 7-amino-4-chloromethyl coumarin leucinamide and/or indigo carmine and mixtures thereof.

14. Method according to one of the preceding claims, wherein a component without photocatalytic coating is used as a negative control 1, and/or wherein a component of which the photocatalytic active surface coating is not activated in step c) by UV light is used as a negative control 2, and/or wherein a component to which no microorganisms are applied in step a) is used as a negative control 3, and/or wherein a component in which the quality and integrity of the photocatalytically active surface coating has already been checked by another test method and found to be sufficient is used as a positive control.

15. Method according to one of preceding claims 11 to 14, wherein the computer to which the test values are fed emits control commands for a sorting device for the components.

16. Method according to one of the preceding claims, wherein the microorganisms and/or the dye according to step d) are washed off from the surface of the components.

17. Device (10) for the non-destructive testing of a photocatalytic surface coating of a ceramic component (20), said device comprising
   a microorganism application station (30) having a storage vessel in which microorganisms are received (30v), and a device for applying the microorganisms (30a) to a test area (90) of a component,
   a dyeing station (40) having a storage vessel (40v) in which one or more dyes are received, and a device for applying the dyes (40a) to the test area (90), and
   an irradiation station (50) having a UV irradiation source (50u) which is arranged above the test area (90),
   a detection station (60) having a detection device arranged above the test area (90), wherein
   the device comprises a transport device (80) on which one or more components (20) is/are transported through the method stations.

18. Device according to claim 17, wherein the microorganism application station and/or the dyeing station and/or the irradiation station and/or the detection station are integrated in a common station.

19. Device according to claim 17 or 18, wherein the temperature of the storage vessel in which the microorganisms are received can be controlled.

20. Device according to one of claims 17 to 19, wherein the device is designed as a device for carrying out a continuous method and/or continuous inline method.

21. Device according to one of claims 17 to 20, which is designed for carrying out the method according to one of claims 1 to 16.

22. Device according to one of claims 17 to 21, wherein, in the dyeing station, the storage vessel, the device for applying the dye solution and/or the dyeing station are formed so as to be impervious to light.

23. Device according to one of claims 17 to 22, wherein the detection device is connected to a computer (70) having evaluation software.

24. Device according to one of claims 16 to 23, wherein the detection device is formed as a fluorometer.

25. Device according to one of claims 16 to 24, wherein the microorganism application station and/or dyeing station additionally have a downstream drying device (30t/40t).

26. Device according to one of claims 16 to 25, wherein the computer is connected to a device for the rejection of faulty components and/or for grouping of the components according to quality classes and/or for acoustic and/or optical signalling and/or labelling of the components.

27. Device according to one of claims 16 to 26, wherein, after the detection station, a cleaning station for cleaning the component of the microorganisms and/or the dye is provided.

**Revendications**

1. Procédé pour un essai non destructif d'un revêtement de surface photocatalytique de composants céramiques, par exemple des tuiles, dans lequel un ou plusieurs composants passent, sur un dispositif de transport, par les étapes suivantes :

   a) application de micro-organismes vivants sur une zone d'essai du revêtement de surface photocatalytique d'un composant céramique,

b) application d'au moins un colorant sur la zone d'essai, le colorant étant configuré de telle sorte que la viabilité des micro-organismes puisse être déterminée par la couleur et/ou l'absorption de la lumière et/ou par la fluorescence du colorant,

c) irradiation de la zone d'essai par une lumière UV, et

d) détection de la couleur et/ou de l'absorption de la lumière et/ou de la fluorescence de la zone d'essai à l'aide d'un dispositif de détection,

dans lequel l'ordre des étapes est de préférence le suivant : a), b), c), d) ou b), a), c), d), ou a), c), b), d), et l'étape a) et l'étape b) et/ou l'étape c) et l'étape d) peuvent chacune être exécutées dans l'ordre indiqué, ou simultanément.

2. Procédé selon la revendication 1, dans lequel il est prévu que le colorant soit couplé aux micro-organismes, et/ou que le colorant soit contenu dans les micro-organismes, et/ou que le colorant et les micro-organismes se présentent en mélange.

3. Procédé selon la revendication 1 ou 2, pour ce qui concerne lequel il s'agit d'un procédé discontinu.

4. Procédé selon l'une des revendications précédentes, dans lequel l'opération d'essai selon la revendication 1 est précédée de la fabrication des composants, de préférence la précède immédiatement, et la fabrication des composants et l'opération d'essai sont mises en oeuvre selon la revendication 1 dans le cadre d'un procédé continu.

5. Procédé selon l'une des revendications 1 à 4, pour ce qui concerne lequel il s'agit d'un procédé en ligne.

6. Procédé selon l'une des revendications précédentes, dans lequel les composants sont soumis à l'essai sur le dispositif de transport par échantillonnage aléatoire.

7. Procédé selon l'une des revendications précédentes, dans lequel, pour ce qui concerne les micro-organismes vivants, il s'agit de bactéries et/ou de champignons et/ou de protozoaires et/ou d'algues.

8. Procédé selon la revendication 7, dans lequel, pour ce qui concerne les micro-organismes vivants, il s'agit d'algues vertes et/ou de cyanobactéries.

9. Procédé selon l'une des revendications précédentes, le procédé étant mis en oeuvre sur un laps de temps de 15 à 120 secondes, de préférence de 15 à 60 secondes, d'une manière particulièrement préférée de 15 à 45 secondes.

10. Procédé selon l'une des revendications précédentes, dans lequel la détection a lieu deux fois, c'est-à-dire à deux instants différents après la mise en oeuvre des étapes a), b) et c).

11. Procédé selon l'une des revendications précédentes, dans lequel, dans l'étape d), la couleur et/ou l'absorption de la lumière et/ou la fluorescence de la zone d'essai sont, sous forme de valeurs d'essai, introduites dans un ordinateur, pour évaluation et/ou pilotage du procédé de fabrication et/ou de la procédure d'essai.

12. Procédé selon l'une des revendications précédentes, dans lequel la plage de longueurs d'onde de la lumière UV dans l'étape c) est choisie dans la plage de 280 à 390 nm, de préférence de 315 à 380 nm.

13. Procédé selon l'une des revendications précédentes, dans lequel, dans l'étape b), on utilise des colorants qui de préférence sont choisis dans le groupe consistant en la chlorophylle, l'orange d'acridine, le DAPI, le CalcoflourWhite, le Sytox, le bleu de bromothymol, le Baclight, le bleu de méthylène, l'orange méthyle, l'iodure de propidium, l'EUB338, le chlorure de cyanoditolyltétrazolium, le Cell Tracker, le Green, le CMAC, le 7-amino-4-chlorométhylcoumarineleucinamide et/ou le carmin d'indigo, et les mélanges de ceux-ci.

14. Procédé selon l'une des revendications précédentes, dans lequel on utilise en tant que témoin négatif 1 un composant sans revêtement photocatalytique, et/ou dans lequel on utilise en tant que témoin négatif 2 un composant dont le revêtement de surface actif photocatalytique n'est pas activé dans l'étape c) par une lumière UV, et/ou dans lequel on utilise en tant que témoin négatif 3 un composant sur lequel, dans l'étape a), aucun micro-organisme n'a été appliqué, et/ou dans lequel on utilise en tant que témoin positif un composant dans lequel la qualité et le caractère complet du revêtement de surface actif photocatalytique ont été déjà contrôlés par une autre procédure d'essai, et se sont avérés suffisants.

**15.** Procédé selon l'une des revendications précédentes 11 à 14, dans lequel l'ordinateur dans lequel on a introduit les valeurs de mesure émet en sortie des instructions de commande pour un dispositif de tri des composants.

**16.** Procédé selon l'une des revendications précédentes, dans lequel les micro-organismes et/ou le colorant sont, après l'étape d), éliminés par lavage de la surface des composants.

**17.** Dispositif (10) pour le contrôle non destructif d'un revêtement de surface photocatalytique d'un composant céramique (20), comprenant :

un poste (30) d'application de micro-organismes, comportant un réservoir dans lequel sont logés des micro-organismes (30v), et un dispositif pour appliquer les micro-organismes (30a) sur une zone d'essai (90) d'un composant,
un poste de coloration (40) comportant un réservoir (40v), dans lequel sont logés un ou plusieurs colorants, et un dispositif pour appliquer les colorants (40a) sur la zone d'essai (90), et
un poste d'irradiation (50), comportant une source de rayonnement UV (50u), qui est disposé au-dessus de la zone d'essai (90),
un poste de détection (60), comportant un dispositif de détection disposé au-dessus de la zone d'essai (90), où le dispositif comprend un dispositif de transport (80), sur lequel un ou plusieurs composants (20) sont transportés en passant par les postes du procédé.

**18.** Dispositif selon la revendication 17, dans lequel le poste d'application des micro-organismes et/ou le poste de coloration et/ou le poste d'irradiation et/ou le poste de détection sont regroupés dans un poste commun.

**19.** Dispositif selon la revendication 17 ou 18, dans lequel le réservoir dans lequel sont logés les micro-organismes peut être porté à l'équilibre de température.

**20.** Dispositif selon l'une des revendications 17 à 19, le dispositif étant configuré comme un dispositif destiné à la mise en oeuvre d'un procédé continu et/ou d'un procédé continu en ligne.

**21.** Dispositif selon l'une des revendications 17 à 20, qui est configuré pour la mise en oeuvre du procédé selon l'une des revendications 1 à 16.

**22.** Dispositif selon l'une des revendications 17 à 21, dans lequel, dans le poste de coloration, le réservoir, le dispositif pour l'application de la solution de colorant et/ou le poste de coloration, sont configurés de façon à être opaques à la lumière.

**23.** Dispositif selon l'une des revendications 17 à 22, dans lequel le dispositif de détection est relié à un ordinateur (70) comportant un logiciel d'évaluation.

**24.** Dispositif selon l'une des revendications 16 à 23, dans lequel le dispositif de détection est configuré sous forme d'un fluorimètre.

**25.** Dispositif selon l'une des revendications 16 à 24, dans lequel le poste d'application des micro-organismes et/ou le poste de coloration comportent en outre en aval un dispositif de séchage (30t/40t).

**26.** Dispositif selon l'une des revendications 16 à 25, dans lequel l'ordinateur est relié à un dispositif pour séparer les composants défectueux et/ou pour regrouper les composants en fonction de classes de qualité et/ou pour assurer une signalisation et/ou un marquage acoustique et/ou optique des composants.

**27.** Dispositif selon l'une des revendications 16 à 26, dans lequel, après le poste de détection, il est prévu un poste de nettoyage destiné à éliminer du composant, par nettoyage, les micro-organismes et/ou le colorant.

Fig. 1

0 sec    15 sec    30 sec    45 sec    60 sec    75 sec

*unbeschichtete Referenzprobe*

*Fig. 2a*

0 sec    15 sec    30 sec    45 sec    60 sec

*$TiO_2$-beschichtete Referenzprobe*

*Fig. 2b*

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102004052764
- WO 02055729 A1
- DE 102004037555 A1